# EUROPEAN PATENT SPECIFICATION

(11) **EP 2 924 122 B1**
(45) Date of publication and mention of the grant of the patent: **23.08.2017**
(21) Application number: 13856298.8
(22) Date of filing: 13.09.2013
(51) Int. Cl.: C12P 7/18, C12N 1/19, C12N 1/21, C12N 15/09, C12N 9/04, C12N 9/02, C12N 9/88, C12N 9/90, C12N 15/52

(54) **1,4-BUTANEDIOL MANUFACTURING METHOD AND MICROORGANISM**
1,4-BUTANDIOL-HERSTELLUNGSVERFAHREN UND MIKROORGANISMUS
PROCÉDÉ DE FABRICATION DE 1,4 BUTANEDIOL ET MICRO-ORGANISME ASSOCIÉ

(30) Priority: 26.11.2012 JP 2012257881
(43) Date of publication of application: 30.09.2015
(73) Proprietor: Showa Denko K.K., Tokyo 105-8518 (JP)
(72) Inventor: AOKI, Hirobumi, Tokyo 105-8518 (JP); KOKIDO, Yuzuru, Tokyo 105-8518 (JP); HASHIMOTO, Yoko, Tokyo 105-8518 (JP); YONEDA, Tadashi, Tokyo 105-8518 (JP)
(74) Representative: Strehl Schübel-Hopf & Partner
(86) International application number: PCT/JP2013/074828
(87) International publication number: WO 2014/080683

(56) References cited:
- EP-A1- 2 924 123
- EP-A1- 2 930 239
- WO-A1-2011/047101
- JP-A- 2012 529 267
- LAN EI ET AL.: 'ATP drives direct photosynthetic production of 1-butanol in cyanobacteria' PROC NATL ACAD SCI USA. vol. 109, April 2012, pages 6018 - 6023, XP055174181
- MADAN VK ET AL.: 'Purification and Properties of NADP-Dependent L(+)-3-Hydroxybutyryl-CoA Dehydrogenase from Clostridium kluyveri' EUR J BIOCHEM. vol. 32, 1973, pages 51 - 56, XP055251675
- SEEDORF H ET AL.: 'The genome of Clostridium kluyveri, a strict anaerobe with unique metabolic features' PROC NATL ACAD SCI USA. vol. 105, 2008, pages 2128 - 2133, XP009119916
- YIM H ET AL.: 'Metabolic engineering of Escherichia coli for direct production of 1,4- butanediol' NAT CHEM BIOL. vol. 7, 2011, pages 445 - 452, XP002706396
- INUI M ET AL.: 'Expression of Clostridium acetobutylicum butanol synthetic genes in Escherichia coli' APPL MICROBIOL BIOTECHNOL. vol. 77, 2008, pages 1305 - 1316, XP008156611

## Description

### BACKGROUND OF THE INVENTION

### 1. Field of the Invention

The present invention relates to a method of manufacturing 1,4-butanediol and a microbe.

### 2. Description of the Related Art

In recent years, attention is paid to a compound manufacturing process with a renewable source as a raw material from the viewpoint of depletion of fossil resource, a countermeasure for global warming, or the like. In particular, a so-called bio-refinery has widely been studied wherein a variety of compounds as raw materials for a polymer or compounds as raw materials for a chemical product are manufactured in biochemical processes with biomass as raw materials.

For compounds that are expected as raw material conversion of biomass, 1,4-butanediol may be used. 1,4-butanediol is widely used as a synthetic raw material for a fine organic chemical product, a monomer unit of a polyester and an engineering plastic or the like, and its market scale is large. Thus, demand for a method of efficiently manufacturing 1,4-butanediol is becoming large in a biochemical process with a renewable source such as biomass or the like as a raw material.

For methods of manufacturing 1,4-butanediol using a biochemical process, for example, methods disclosed in Patent documents 1 and 2 and Non-patent document 1 may be used.

[Patent document 1] Japanese Patent No. 4380704 specification
[Patent document 2] International Publication No. 2008/115840 official gazette
[Non-patent document 1] Harry Yim et al., Metabolic engineering of Escherichia coli for direct production of 1,4-butanediol, Nature Chemical Biology, 7, 445-452 (2011) .

### SUMMARY OF THE INVENTION

However, the methods disclosed in Patent documents 1 and 2 and Non-patent document 1 are complicated processes.

For a problem as described above, there is provided a new method of manufacturing 1,4-butanediol capable of economically obtaining 1,4-butanediol.

The present invention includes the following.
[1] A method of manufacturing 1,4-butanediol, using a microbe and/or a culture thereof, by an enzyme reaction system that uses acetoacetyl-CoA reductase and enoyl-CoA hydratase, via acetoacetyl-CoA, 3-hydroxybutyryl-CoA and crotonyl-CoA in this order,
   wherein the acetoacetyl-CoA reductase is (R)-3-hydroxybutyryl-CoA selective acetoacetyl-CoA reductase and the enoyl CoA reductase is (R)-3-hydroxybutyryl-CoA selective enoyl-CoA hydratase, and
   wherein the microbe includes
   (1) a gene that codes the (R)-3-hydroxybutyryl-CoA selective acetoacetyl-CoA reductase, and
   (2) a gene that codes the (R)-3-hydroxybutyryl-CoA selective enoyl-CoA hydratase.
[2] The method of manufacturing 1,4-butanediol according to [1],
   wherein the (R)-3-hydroxybutyryl-CoA selective acetoacetyl-CoA reductase is acetoacetyl-CoA reductase (EC number: 1.1.1.36), and
   wherein the (R)-3-hydroxybutyryl-CoA selective enoyl-CoA hydratase is enoyl-CoA hydratase (EC number: 4.2.1.55 or EC number: 4.2.1.119).
[3] The method of manufacturing 1,4-butanediol according to [1],
   wherein a gene that codes the (R)-3-hydroxybutyryl-CoA selective acetoacetyl-CoA reductase is any one of undermentioned genes (a) to (c), and
   wherein a gene that codes the (R)-3-hydroxybutyryl-CoA selective enoyl-CoA hydratase is any one of undermentioned genes (d) to (f):
   (a) a gene that has a base sequence of sequence number 3;
   (b) a gene that has a base sequence such that one or more bases are deleted, substituted, or added in a base sequence of sequence number 3, wherein the gene has a base sequence with an identity greater than or equal to 90% with respect to the base sequence of sequence number 3;
   (c) a gene that hybridizes with a gene that has a base sequence complementary with a gene that has a base sequence described in sequence number 3 on a stringent condition;
   (d) a gene that has a base sequence of sequence number 5;
   (e) a gene that has a base sequence such that one or more bases are deleted, substituted, or added in a base sequence of sequence number 5, wherein the gene has a base sequence with an identity greater than or equal to 90% with respect to the base sequence of sequence number 5;
   (f) a gene that hybridizes with a gene that has a base sequence complementary with a gene that has a base sequence described in sequence number 5 on a stringent condition.
[4] The method of manufacturing 1,4-butanediol according to [1],
   wherein the microbe further includes,
   (1) a gene that codes vinylacetyl-CoA delta-isomerase (EC number: 5.3.3.3),
   (2) a gene that codes 4-hydroxybutyryl-CoA dehydratase (EC number: 4.2.1.120), and/or
   (3) a gene that codes acyl-CoA reductase (EC number: 1.2.1.10).
[5] The method of manufacturing 1,4-butanediol according to [1],
   wherein the microbe is Escherichia coli, a yeast, a corynebacterium or a clostridial bacteria.
[6] A microbe that manufactures 1,4-butanediol by an enzyme reaction system that uses acetoacetyl-CoA reductase and enoyl-CoA hydratase, via acetoacetyl-CoA, 3-hydroxybutyryl-CoA and crotonyl-CoA in this order,
   wherein the acetoacetyl-CoA reductase is (R)-3-hydroxybutyryl-CoA selective acetoacetyl-CoA reductase and the enoyl CoA reductase is (R)-3-hydroxybutyryl-CoA selective enoyl-CoA hydratase, and
   wherein the microbe includes
   (1) a gene that codes the (R)-3-hydroxybutyryl-CoA selective acetoacetyl-CoA reductase, and
   (2) a gene that codes the (R)-3-hydroxybutyryl-CoA selective enoyl-CoA hydratase.
[7] The microbe according to [6],
   wherein the microbe is Escherichia coli, a yeast, a corynebacterium or a clostridial bacteria.
A method of economically manufacturing 1,4-butanediol is provided.

### BRIEF DESCRIPTION OF THE DRAWINGS

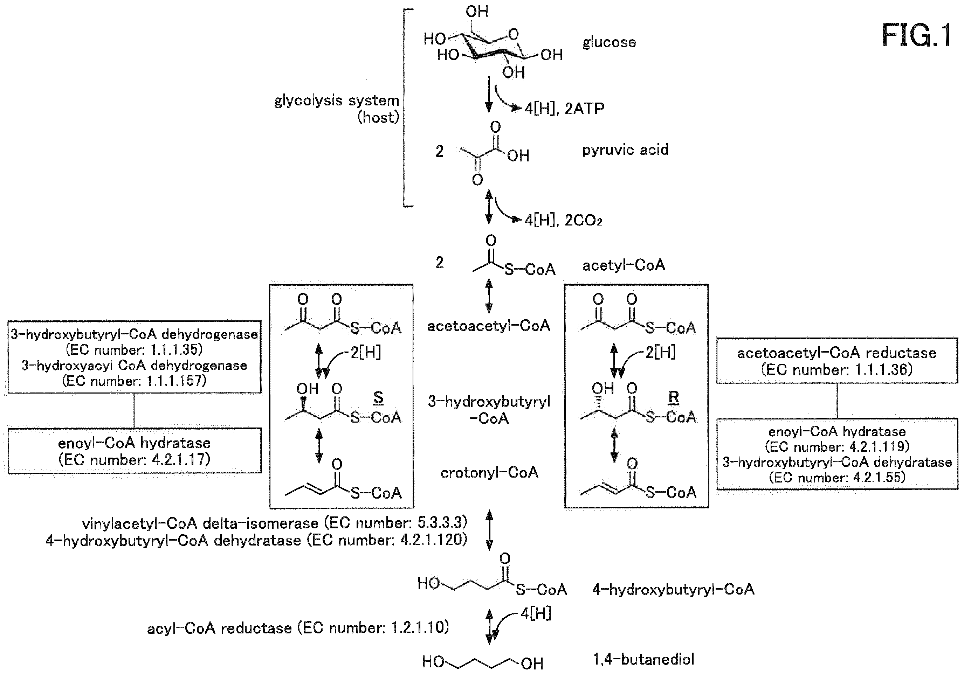

Fig. 1 is a view illustrating an example of an enzyme system of a method of manufacturing 1,4-butanediol of an embodiment.

### DETAILED DESCRIPTION OF THE PREFERRED EMBODIMENTS

The present invention will be described in detail below. Here, in this specification, "CoA" means "coenzyme A". Furthermore, "%" indicates "% by mass" unless otherwise described. "ppm" is mass basis.

One of features of the present invention is a method of using a microbe or a culture thereof capable of selectively manufacturing 1,4-butanediol with high productivity. Thus, a microbe that has an enzyme system is used in which 1,4-butanediol is manufactured via acetoacetyl-CoA, 3-hydroxybutyryl-CoA, crotonyl-CoA and 4-hydroxybutyryl-CoA in this order. More specifically, a microbe that has an enzyme system in which 1,4-butanediol is generated via acetoacetyl-CoA, isomerization between 3-hydroxybutyryl-CoA and 4-hydroxy CoA and reduction of 4-hydroxybutyryl-CoA.

Hereinafter, a feature of a microbe, a method of manufacturing the microbe, a method of using the microbe (in other words, a method of manufacturing 1,4-butanediol), a method of obtaining the manufactured 1,4-butanediol or the like, that is used in this embodiment, is explained.

### (Host microbe)

It is possible to introduce various genes, which will be explained later, to the host microbe used in this embodiment and a gene recombination technique can be applied to the host microbe, for example. Specifically, for example, the host microbe is one capable of being transformed into a microbe in which an enzyme system is added by which 1,4-butanediol is manufactured via acetoacetyl-CoA, 3-hydroxybutyryl-CoA, crotonyl-CoA and 4-hydroxybutyryl-CoA in this order under an appropriate culturing condition, which will be explained later, in addition to an enzyme system that is originally possessed by the host microbe.

In this embodiment, an example of the above mentioned host microbe to which various genes can be introduced is not particularly limited as long as a gene recombination technique can be applied to the host microbe. From the viewpoint of industrial availability, for a specific example, Escherichia coli, a yeast, a coryneform bacteria or a clostridial bacteria may be used. For a yeast, Saccharomyces cerevisiae, Schizosaccharomyces pombe, Kluyveromyces lactis, Kluyveromyces marxianus or the like may be used. For a coryneform bacteria, Corynebacterium glutamicum, Corynebacterium efficiens, Brevibacterium divaricatum, Brevibacterium saccharolyticum, Brevibacterium immariophilum, Brevibacterium lactofermentum, Brevibacterium roseum, Brevibacterium flavum, Brevibacterium thiogenitalis, Corynebacterium acetoacidophilum, Corynebacterium acetoglutamicum, Corynebacterium callunae, Corynebacterium lilium, Corynebacterium mellassecola, Microbacterium ammoniaphilum or the like may be used. For a clostridial bacteria, Clostridium kluyveri, Clostridium acetobutylicum, Clostridium aminobutyricum, Clostridium beijerinckii, Clostridium saccharoperbutylacetonicum or the like may be used. Among these, it is preferable to use Escherichia coli, Saccharomyces cerevisiae, Schizosaccharomyces pombe or Corynebacterium glutamicum, because transformation thereof is easy, and it is more preferable to use Escherichia coli.

Further, the transformation microbe of the embodiment may be used in various embodiments including a cultured microbial cell itself and a culture thereof. The culture of the microbe of the embodiment includes, specifically, a suspended material of a cultured microbial cell by a medium such as a culture medium, buffer or the like, a cell-free extract from a cultured microbial cell, and further a processed material such as a material obtained by condensing, purifying or extracting a component that catalyzes the reaction from the cell-free extract or the like. The culture of the microbe of the embodiment further includes a material in which a processed material of the microbe is immobilized on an insoluble carrier. For such an immobilized carrier, a compound that forms a water-insoluble solid content containing the above described microbial cell or its processed material such as polyacrylamide, polyvinyl alcohol, poly(N-vinylformamide), polyallylamine, polyethyleneimine, methylcellulose, glucomannan, alginate, carrageenan or the like, further, a copolymer or a crosslinked material thereof or the like may be used. A kind of these may be used or a mixture of two or more kinds of these may be used. Further, a material in which the microbe or its extracting solution or extracting component is held on an object previously formed as a solid material such as an activated carbon, porous ceramics, a glass fiber, a porous polymer plastic body, a nitrocellulose film or the like may be used as the culture of the microbe as well.

### (Transformation microbe)

The host microbe used in this embodiment is a host microbe into which various genes, which will be explained later, can be introduced, and, for example, a gene recombination technique can be applied to the host microbe. Specifically, the transformation microbe further has each enzyme system of an enzyme reaction system by which at least 1,4-butanediol can be manufactured via acetoacetyl-CoA, 3-hydroxybutyryl-CoA, crotonyl-CoA and 4-hydroxybutyryl-CoA in this order, in addition to an enzyme system that is originally possessed by the host microbe. Hereinafter, a gene that codes each enzyme system is explained.

### (Gene that codes (R)-3-hydroxybutyryl-CoA selective acetoacetyl-CoA reductase)

In this specification, a "gene that codes (R)-3-hydroxybutyryl-CoA selective acetoacetyl-CoA reductase" means a gene that codes an enzyme capable of catalyzing a reaction in which acetoacetyl-CoA is reduced as a substrate (or may be an intermediate or a precursor) to generate (R)-3-hydroxybutyryl-CoA.

For a specific example of the gene that codes the enzyme ((R)-3-hydroxybutyryl-CoA reductase) capable of catalyzing the reaction in which acetoacetyl-CoA is reduced as the substrate (or may be the intermediate or the precursor) to generate (R)-3-hydroxybutyryl-CoA, although not particularly limited, a gene, a homolog thereof or the like that codes acetoacetyl-CoA reductase (EC number: 1.1.1.36) may be used.

### (Gene that codes (R)-3-hydroxybutyryl-CoA selective enoyl-CoA hydratase)

In this specification, a "gene that codes (R)-3-hydroxybutyryl-CoA selective enoyl-CoA hydratase" means a gene that codes an enzyme capable of catalyzing a reaction in which (R)-3-hydroxybutyryl-CoA is dehydrated as a substrate (or may be an intermediate or a precursor) to generate crotonyl-CoA.

For a specific example of the gene that codes the enzyme capable of catalyzing the reaction in which crotonyl-CoA is generated from (R)-3-hydroxybutyryl-CoA, used in this embodiment, although not particularly limited, a gene, a homolog thereof or the like that codes enoyl-CoA hydratase (EC number: 4.2.1.55 (3-hydroxybutyryl-CoA dehydratase) or EC number: 4.2.1.119) may be used. For the detail of the above mentioned enzyme, Non-patent documents such as "Fukui, T., Shiomi, N. and Doi, Y., Expression and characterization of (R)-specific enoyl coenzyme A hydratase involved in polyhydroxyalkanoate biosynthesis by Aeromonas caviae, J. Bacteriol. 180 (1998), 667-673.", "Moskowitz, G.J. and Merrick, J.M., Metabolism of poly-β-hydroxybutyrate. II. Enzymatic synthesis of D-(-)-β-hydroxybutyryl coenzyme A by an enoyl hydrase from Rhodospirillum rubrum, Journal Biochemistry 8 (1969), 2748-2755." or the like may be referred.

### (Gene that codes vinylacetyl-CoA delta-isomerase)

In this embodiment, a gene that codes vinylacetyl-CoA delta-isomerase is not particularly limited as long as it is a gene that codes an enzyme capable of catalyzing a reaction in which vinylacetyl-CoA is generated by transitioning olefin of crotonyl-CoA.

For a specific example of the enzyme capable of catalyzing the above mentioned reaction, although not limited, vinylacetyl-CoA delta-isomerase (EC number: 5.3.3.3), a homolog thereof or the like may be used. For the detail of the above mentioned enzyme, for example, a Non-patent document such as "Fermentation of 4-aminobutyrate by Clostridium aminobutyricum: cloning of two genes involved in the formation and dehydration of 4-hydroxybutyryl-CoA, Archives of Microbiology, 174(3) 189-199 (2000)." or the like may be referred.

### (Gene that codes 4-hydroxybutyryl-CoA dehydratase)

In this embodiment, a gene that codes 4-hydroxybutyryl-CoA dehydratase is not particularly limited as long as it is a gene that codes an enzyme that catalyzes a reaction in which vinylacetyl-CoA is hydrated to generate 4-hydroxybutyryl-CoA.

For a specific example of the above mentioned enzyme capable of catalyzing the reaction, although not limited, 4-hydroxybutyryl-CoA dehydratase (EC number: 4.2.1.120), a homolog thereof or the like may be used. For the detail of the above described enzyme, for example, a Non-patent document such as "Fermentation of 4-aminobutyrate by Clostridium aminobutyricum: cloning of two genes involved in the formation and dehydration of 4-hydroxybutyryl-CoA, Archives of Microbiology, 174(3) 189-199 (2000)." or the like may be referred. Here, an example in this Non-patent document relates to a multienzyme of 4-hydroxybutyryl-CoA dehydratase and the above described vinylacetyl-CoA delta-isomerase (EC number: 5.3.3.3) and a gene that codes thereof. However, as long as functions of each enzyme can be appropriately provided, each enzyme or an individual gene that codes a catalytic subunit protein may be used.

### (Gene that codes acyl-CoA reductase)

In this embodiment, a gene that codes acyl-CoA reductase is not particularly limited as long as it codes an enzyme capable of catalyzing a reaction in which 4-hydroxybutyryl-CoA is reduced and 4-hydroxybutanal is generated.

For a specific example of the above described enzyme capable of catalyzing the reaction, although not limited, aldehyde dehydrogenase (acylation) (EC number: 1.2.1.10) or a homolog thereof may be used. Here, although the obtained 4-hydroxybutanal is led to 1,4-butanediol by alcohol reductase that is normally possessed by the host, which will be explained later, a gene that codes alcohol reductase may be additionally expressed to manufacture 1,4-butanediol.

### (Method of manufacturing microbe)

Introduction of a gene to a host microbe may be performed by appropriately combining various known methods, for example, such as a method based on restriction enzyme / ligation, an In-Fusion cloning method or the like, such that the above described gene or its part is connected to an appropriate vector and the obtained recombinant vector is introduced into the host so that a purposed gene can be expressed. Alternatively, introduction of a gene to a host microbe may be performed by inserting a target gene or its part at an arbitrary site of a genome by a homologous recombination. "Part" indicates a part of each gene capable of expressing a protein coded by the respective gene when being introduced into the host. In this invention, a gene includes DNA and RNA, and is preferably DNA.

For a vector that connects the gene, it is not particularly limited as long as it is duplicable by a host, and for example, a plasmid, a phage, a cosmid or the like that is used for transgenesis in an Escherichia coli may be used. For a plasmid, for example, pHSG398, pUC18, pBR322, pSC101, pUC19, pUC118, pUC119, pACYC117, pBluescript II SK(+), pET17b, pETDuet-1, pACYCDuet-1, pCDFDuet-1, pRSFDuet-1, pCOLADuet-1 or the like may be used. For a phage, for example, λgt10, Charon 4A, EMBL-, M13mp18, M13mp19 or the like may be used. Some of them are commercially available and a commercial item (kit) may be used as it is in accordance with its procedures manual, or after appropriately altering.

In the above described vector, an appropriate expression promoter may be connected at upstream of a gene such that the inserted gene is surely expressed. The expression promoter to be used is not particularly limited and is appropriately selected by a person skilled in the art in accordance with the host. For example, T7promoter, lacpromoter, trppromoter, trcpromoter or λ-PLpromoter that is used for expressing a foreign gene in Escherichia coli, a Nar promoter region of nitrate reductase gene narGHJI operon that relates to nitrate respiration derived from Escherichia coli, or a promoter region of Frd gene that is nitrate reductase gene of Escherichia coli may be used.

Further, for some cases, it is preferable that the original gene of the host microbe is disrupted so that the gene is not expressed. For a method of disrupting the gene, known methods that are used for disrupting a gene in Escherichia coli may be used. Specifically, a method that is used for manufacturing a knockout cell or the like in this technical field such as a method of disrupting a gene (gene targeting method) using a vector (targeting vector) that generates a homologous recombination at an arbitrary position of a target gene, a method (gene trapping method) in which a trap vector (reporter gene that does not have promoter) is inserted at an arbitrary position of a target gene to disrupt the gene and deactivate its function, a method of combination thereof or the like may be used.

The position at which the homologous recombination is generated or the position to which the trap vector is inserted is not particularly limited as long as it is a position at which modification to deactivate the expression of the target gene to be disrupted, but preferably, is a transcriptional control region.

Further, for a method of introducing the vector to the host, although not particularly limited, for example, a method of using calcium ion, a protoplast method, an electroporation method or the like that is generally used for introducing a vector to Escherichia coli may be used.

A method of inserting a target gene to an arbitrary position on a genome by homologous recombination may be performed by inserting the target gene to a homogenous sequence of a sequence on the genome with a promoter and introducing the nucleic acid fragment to a cell by electroporation to generate homologous recombination. When introducing to the genome, a stock may be selected by which homologous recombination easily occurred when using a nucleic acid fragment to which the target gene and a drug-resistant gene are connected. Further, the target gene may be introduced by homologous recombination by inserting a gene to which a drug-resistant gene and a gene that becomes fatal under a specific condition are connected to the genome by the above described method by homologous recombination, and thereafter, substituting the drug-resistant gene and the gene that becomes fatal under a specific condition.

Further, for a method of selecting a recombinant microbe in which the target gene is introduced, although not particularly limited, it is preferably to use a method by which only a recombinant microbe in which the target gene is introduced can be easily selected.

### (Method of using microbe for manufacturing 1,4-butanediol, culturing condition and method of obtaining obtained 1,4-butanediol)

Fig. 1 illustrates an example of an enzyme system of the method of manufacturing 1,4-butanediol of the embodiment. In this embodiment, 1,4-butanediol can be obtained by using a culture in which the above described series of genes are expressed in a microbial body by means of transformation or the like. Here, each of the genes, individually or as a series of cluster, transforms the host microbe by being inserted into an arbitrarily vector. By culturing the obtained transformed body in a medium with an appropriate carbon source, for example, glucose, each of the genes is expressed. For a gene that can be constitutively expressed in a host, the gene can be expressed by culturing the transformed body in the medium. On the other hand, when each of the genes is constituted under a control of a regulator that is provided on a vector, each of the genes that codes is expressed by adding a deriving substrate to transfer to a deriving environment. Here, culturing in this embodiment includes all of culturing conditions for normal microbe culturing, and further, a step of culturing means that culturing with sufficient period and condition for the microbe to manufacture 1,4-butanediol.

The present inventors have found that the productivity of 1,4-butanediol is significantly improved by using a gene that codes a specific kind of enzyme.

In order to express a gene that codes (R)-3-hydroxybutyryl-CoA selective acetoacetyl-CoA reductase (EC number: 1.1.1.36), a microbe or a culture thereof is used in which a gene that codes enoyl-CoA hydratase (EC number: 4.2.1.119) or 3-hydroxybutyryl-CoA dehydratase (EC number: 4.2.1.55) is expressed as well.

Further, in the method of manufacturing 1,4-butanediol of the embodiment, it is preferable that a microbe or a culture thereof is used in which a gene that codes vinylacetyl-CoA delta-isomerase (EC number: 5.3.3.3), a gene that codes 4-hydroxybutyryl-CoA dehydratase (EC number: 4.2.1.120) and/or a gene that codes acyl-CoA reductase (EC number: 1.2.1.10) is expressed as well, in addition to the microbe in which the above described genes are expressed by means of transformation or the like. It is preferable to combine all of or a part of these genes, as the productivity of 1,4-butanediol is further improved.

As such, as the method of manufacturing 1,4-butanediol of the embodiment uses a gene that codes a specific enzyme, 1,4-butanediol can be manufactured with high productivity.

Here, in this embodiment, a method of supplying acetoacetyl-CoA that is a substrate of acetoacetyl-CoA reductase and provides 3-hydroxybutyryl-CoA is not particularly limited. For example, a method or the like may be used in which acetyl-CoA that is derived from and supplied from an arbitrary carbon source used for culturing such as sugar such as glucose by a glycolysis system possessed by a host, which will be explained later. At this time, under a condition in which acetyl-CoA is supplied, acetoacetyl-CoA can be supplied using a gene or a homolog thereof that codes β-ketothiolase (EC number: 2.3.1.9) by which one molecule of CoA is desorbed from two molecules of acetyl-CoA to supply acetoacetyl-CoA.

Alternatively, acetoacetyl-CoA may be supplied using a gene or a homolog thereof that codes acetoacetyl-CoA synthase (EC number: 2.3.1.194) that catalyzes a reaction that irreversibly produces acetoacetyl-CoA using acetyl-CoA and malonyl-CoA as substrates (or may be an intermediate or a precursor). For the detail of the acetoacetyl-CoA synthase, for example, a Non-patent document such as "Unprecedented acetoacetyl-coenzyme A synthesizing enzyme of the thiolase superfamily involved in the mevalonate pathway., Proc. Natl. Acad. Sci., U. S. A. 107, 11265 - 11270 (2010)" or the like may be referred to.

A homolog referred to in the embodiment includes an ortholog and a paralog. An ortholog refers to a set of corresponding genes among species generated from a gene of a common ancestor by means of speciation and enzymes obtained from such genes. A paralog refers to a set of corresponding genes among species generated by means of not speciation but gene duplication in an identical species and enzymes obtained from such genes. A homolog refers to genes that have an identity in sequences thereof, regardless of an ortholog or a paralog, and enzymes obtained from such genes.

More specifically, a homolog (gene) in this embodiment is a gene that has a base sequence with an identity greater than or equal to 90%, preferably an identity greater than or equal to 95%, and more preferably, a gene that is completely identical to such a gene or wherein one or several bases thereof are deleted, substituted, or added.

Furthermore, a homolog gene includes a gene that hybridizes with a gene that has a base sequence complementary with a target gene on a stringent condition. Specifically, it is possible to acquire a gene or an enzyme that is obtained by transformation caused by such a gene, by applying a homology retrieval program (for example, BLAST or FASTA) to a publicly-known data base, or based on an ordinary method such as hybridization, polymerase chain reaction (PCR) or the like under a stringent condition that uses a probe that is composed of at least a part of an identified gene (DNA that is composed of a base sequence complementary with DNA that is composed of a base sequence of such a gene). Furthermore, it is possible for a person skilled in the art to execute self-design by substituting a base sequence or the like. Here, for a stringent condition referred herein, for example, a condition for executing hybridization as described in a Non-patent document of "Molecular Cloning - A LABORATORY MANUAL THIRD EDITION (Joseph Sambrook, David W. Russell., Cold Spring Harbor Laboratory Press)". Specifically, a hybridizing condition is to be retained with a probe in a solution that includes 6 x SSC (a composition of 1 x SSC: 0.15 M of sodium chloride, 0.015 M of sodium citrate, pH: 7.0), 0.5% of SDS, 5 x Denhardt's solution and 100 mg/mL of herring sperm DNA, at a constant temperature of 65 °C for 8 - 16 hours.

### (Culturing method)

For example, a reaction in the present invention is most conveniently achieved in such a manner that a transformed body is cultured in a nutritive medium such as an LB medium at a temperature of 15 °C - 40 °C, desirably 18 °C - 37 °C for about 24 hours, subsequently implanted to a medium with a normal carbon source, for example, a carbon source that is 0.01 - 50%, desirably 0.1 - 30%, of glucose, and continuously cultured at a similar temperature for about 1 hour - 200 hours, wherein 1,4-butanediol is stored in a culture solution in such a process. Furthermore, a carbon source may be added continuously or intermittently depending on consumption of a carbon source that is caused by proliferation of a bacteria or proceeding of a reaction, and in such a case, a concentration of a carbon source in a reaction fluid is not limited to one described previously.

For a medium carbon source for culturing a microbe, it is possible to use, for example, a saccharide such as glucose, sucrose, fructose or the like, a polyol such as glycerol or the like, an organic substance such as ethanol, acetic acid, citric acid, succinic acid, lactic acid, benzoic acid, fatty acid or the like, or an alkali metal salt thereof, an aliphatic hydrocarbon such as n-paraffin or the like, an aromatic hydrocarbon or a natural organic substance such as peptone, meat extract, fish extract, soybean flour, bran or the like, for example, singly or in combination thereof, at a concentration of normally 0.01% - 30%, desirably about 0.1% - 20%.

For a medium nitrogen source for culturing a microbe, it is possible to use, for example, an inorganic nitrogen compound such as ammonium sulfate, ammonium phosphate, sodium nitrate, potassium nitrate or the like, a nitrogen-containing organic substance such as urea, uric acid or the like, or a natural organic substance such as peptone, meat extract, fish extract, soybean flour or the like, singly or in combination thereof, at a concentration of normally 0.01% - 20%, desirably about 0.1% - 10%.

Moreover, it is possible to add a phosphate such as potassium dihydrogen phosphate or the like, or a metal salt such as magnesium sulfate, ferrous sulfate, calcium acetate, manganese chloride, copper sulfate, zinc sulfate, cobalt sulfate, nickel sulfate or the like, in accordance with necessity, for growth of a bacteria or improvement of an enzyme activity. A concentration for addition thereof is different depending on a culturing condition, and normally, is 0.01% - 5% for a phosphate, 10 ppm - 1% for a magnesium salt, or about 0.1 ppm - 1,000 ppm for other compounds. Furthermore, for a source of supply for a vitamin, an amino acid, a nucleic acid or the like, it is possible to add, for example, about 1 ppm - 100 ppm of yeast extract, casamino acid, or a yeast nucleic acid, depending on a selected medium, for growth of a bacteria or improvement of an enzyme activity.

It is desirable to adjust a pH of a medium to 4.5 - 9, desirably 5 - 8. Furthermore, in order to reduce an impurity in a reaction fluid and simplify subsequent fractionation of a product, it is useful to fractionate a bacterial body that is preliminarily cultured in a medium as described previously from a culture solution by a method such as centrifugation or membrane filtration, and again suspend and react in water that includes a reaction raw material, physiological saline, a buffer that has a pH adjusted to be comparable to a pH for culturing and composed of phosphoric acid, acetic acid, boric acid, tris(hydroxymethyl)aminomethane or the like and a salt thereof, or the like. Although a pH during a reaction is able to be normally retained in a case where a buffer with a sufficient concentration is used, it is desirable to be adjusted appropriately by using sodium hydroxide, ammonia or the like so as to provide a similar pH in a case where deviation from a pH as described above is caused by proceeding of a reaction.

When a reaction rate is lowered due to 1,4-butanediol stored in a reaction fluid, a method is preferable that adds water, physiological saline, a reaction buffer or the like, into a reaction fluid depending on a concentration of a product to execute continuous dilution thereof. Furthermore, at a point of time when a reaction rate is lowered, a bacteria is fractionated and a supernatant is recovered as a product solution, wherein a fractionated bacteria is again returned to a solution or suspension that includes a reaction raw material and thereby it is possible to recover a reaction rate. It is possible to execute such an operation continuously or even batch-wise by using a centrifuge, a separation film or the like.

It is possible to execute separation, recovery and purification of 1,4-butanediol produced in a reaction fluid, at a point of time when an amount of produced 1,4-butanediol reaches a substantial amount, by using a general means of separation, recovery, and purification of an organic compound, after a bacterial body is eliminated from such a reaction fluid by means of centrifugation or for such a reaction fluid directly. For example, extraction from a filtrate wherein a bacterial body and the others are eliminated from a culture solution is executed by using an appropriate organic solvent. Such an extract is directly distilled away, is further extracted again with an appropriate solvent, purified by using silica gel chromatography or the like, is subjected to multistage distillation or the like, and thereby, 1,4-butanediol is obtained at a high purity.

### (Examples and comparative examples)

The present invention is further explained in detail by explaining examples.

Table 1 illustrates assumed reaction processes, enzymes that catalyze the respective reaction processes, and used genes that code the respective enzymes. Here, the sequence number illustrated for each of the genes corresponds to the sequence number in a sequence listing.

**[Table 1]**

| REACTION PROCESS | ENZYME THAT CATALYZES RESPECTIVE REACTION | USED GENE | |
|---|---|---|---|
| | | EXAMPLES 1-2 AND COMPARATIVE EXAMPLES 1-2 | |
| | | GENE OF EXAMPLE | DERIVATION |
| (1) | *β* -ketothiolase, acetyl-CoA acetyltransferase, acetoacetyl-CoA synthase (EC number: 2.3.1.9) | SEQUENCE NUMBER 1 | atoB (Escherichia coli K12 stock derived sequence, artificial composition) |
| process to transform acetyl-CoA to acetoacetyl-CoA | | | |
| (2) | 3-hydroxybutyryl-CoA dehydrogenase (EC number: 1.1.1.35), 3-hydroxyacyl CoA dehydrogenase (EC number: 1.1.1.157) | SEQUENCE NUMBER 2 | hbd (Clostridium kluyveri DSM555 derived sequence, artificial composition) |
| process to transform | | | |
| acetoacetyl-CoA to 3-hydroxybutyryl-CoA | acetoacetyl-CoA reductase (EC number: 1.1.1.36) | SEQUENCE NUMBER 3 | PhaB (Ralstonia eutropha H16 derived, artificial composition) |
| (3) | enoyl-CoA hydratase (EC number: 4.2.1.17) | SEQUENCE NUMBER 4 | crt (Clostridium acetobutylicum ATCC 824 derived sequence, artificial composition) |
| process to transform 3-hydroxybutyryl-CoA to crotonyl-CoA | | | |
| | enoyl-CoA hydratase (EC number: 4.2.1.119), 3-hydroxybutyryl-CoA dehydratase (EC number: 4.2.1.55) | SEQUENCE NUMBER 5 | phaJ (Aeromonas caviae derived sequence, artificial composition) |
| (4) | vinylacetyl-CoA delta-isomerase (EC number: 5.3.3.3) + 4-hydroxybutyryl-CoA dehydratase (EC number: 4.2.1.120) | SEQUENCE NUMBER 6 | provided by the inventors (artificial composition) |
| process to transform crotonyl-CoA to 4-hydroxybutyryl-CoA | | | |
| (5) | acyl-CoA reductase (EC number: 1.2.1.10) | SEQUENCE NUMBER 7 | ald (Clostridium beijerinckii NRRL B592 derived sequence, artificial composition) |
| process to transform 4-hydroxybutyryl-CoA to 1,4-butanediol | | | |

### (Example 1) (Reference only)

A blunt end fragment was prepared by an ordinary method in which sequences corresponding to 15 base pairs at an upstream side and at a downstream side including upstream side CAT and downstream side ATG of an NdeI site in multi-cloning sites of an expression vector pET17b (produced by Novagen, Inc.), respectively, were added to an upstream and a downstream, at a 5'-end side and a 3'-end side, of a gene sequence indicated by sequence number 1. Ligation of the fragment and a fragment obtained by performing an NdeI process on pET17b (produced by Novagen, Inc.) was executed by In-Fusion HD Cloning Kit (produced by TAKARA BIO INC.) to obtain plasmid pETBD1.

A gene sequence indicated by sequence number 2 was inserted into pET17b targeting at an NdeI site by a method similar to that of pETBD1 to obtain plasmid pETBD2 containing sequence 2.

An EcoRI site derived from multi-cloning sites of pET17b that was positioned at a downstream of a termination codon of sequence 1 of pETBD1 was cleaved by an enzyme process to prepare a ring-opened fragment of pETBD1. Next, a fragment was prepared by PCR in which sequences corresponding to upstream side 15bp and downstream side 15bp including the EcoRI site of the pETBD1 were added at an upstream and a downstream of a region of the sequence 2 and its upstream region including T7 promoter derived from pET17b of the pETBD2. Ligation of two obtained fragments was executed by an In-Fusion HD Cloning Kit to obtain plasmid pETBD1-2 containing sequences 1 and 2.

Hereinafter, similarly, by targeting sequences further downstream of sequence 2 of pETBD1-2, sequence number 4, 6 and 7 were added in this order to obtain plasmid pETBD1-2-4-6-7. Here, when adding the sequences, opening of plasmid to which sequences were inserted was executed, by cleavage using a restriction enzyme that does not cleave the inserted when an appropriate restriction enzyme site for the restriction enzyme existed on a vector, or by an inverse PCR from a site to be inserted when such a site did not exist. By pETBD1-2-4-6-7, Escherichia coli pETBD1-2-4-6-7/JM109 (DE3) that was transformed from an Escherichia coli JM109 (DE3) stock was obtained.

### (Comparative example 1)

By a method similar to that of Example 1, Escherichia coli pETBD1-2-5-6-7/JM109 (DE3) that was transformed by plasmid pETBD1-2-5-6-7 in which the gene of sequence 4 of plasmid pETBD1-2-4-6-7 was substituted by a gene indicated by sequence number 5 that codes an enzyme corresponding to the enzyme that catalyzes the process.

### (Example 2)

By a method similar to that of Example 1, Escherichia coli pETBD1-3-5-6-7/JM109 (DE3) that was transformed by plasmid pETBD1-3-5-6-7 in which the genes of sequences 2 and 4 of plasmid pETBD1-2-4-6-7 were substituted by genes indicated by sequence numbers 3 and 5 that code enzymes corresponding to the enzymes that catalyze each of the processes.

### (Comparative example 2)

By a method similar to that of Example 1, Escherichia coli pETBD1-3-4-6-7/JM109 (DE3) that was transformed by plasmid pETBD1-3-4-6-7 in which the gene of sequence 5 of plasmid pETBD1-3-5-6-7 was substituted by a gene indicated by the sequence number 4 that codes an enzyme corresponding to the enzyme that catalyzes the process (comparative example 2).

Each of the obtained transformed bodies was aerobically cultured in 5 mL of an LB medium that contained 100 mg/L of ampicillin at 37 °C for 12 hours. 0.1 mL of a culture solution was implanted into 5 mL of an LB medium that contained 1% of glucose, 100 mg/L of ampicillin, and 0.2 mM of IPTG and aerobically cultured at 30 °C for 48 hours. A supernatant of the culture solution was subjected to high performance liquid chromatography (HPLC: column; Shodex SH-1011 (produced by Showa Denko K. K.), column temperature: 60 °C, eluent: 25 mM sulfuric acid aqueous solution, flow rate 0.6 mL/min, detection: differential refraction detector). Table 2 illustrates a relationship between genes that compose plasmids of the used transformed body and the amount of 1,4-butanediol generated in a culturing solution.

**[Table 2]**

| REACTION PROCESS | USED GENE (SEQUENCE NUMBER) | | | |
|---|---|---|---|---|
| | COMPARATIVE EXAMPLE 1 | EXAMPLE 1* | COMPARATIVE EXAMPLE 2 | EXAMPLE 2 |
| (1) | 1 | 1 | 1 | 1 |
| process to transform acetyl-CoA to acetoacetyl-CoA | | | | |
| (2) | 2 | 2 | 3 | 3 |
| process to transform acetoacetyl-CoA to 3-hydroxybutyryl-CoA | | | | |
| (3) | 5 | 4 | 4 | 5 |
| process to transform 3-hydroxybutyryl-CoA to crotonyl-CoA | | | | |
| (4) | 6 | 6 | 6 | 6 |
| process to transform crotonyl-CoA to 4-hydroxybutyryl-CoA | | | | |
| (5) | 7 | 7 | 7 | 7 |
| process to transform 4-hydroxybutyryl-CoA to 1,4-butanediol | | | | |
| Amount of manufactured 1,4-butanediol (mg/L) | ND | 58 | ND | 106 |

| | | | | |
|---|---|---|---|---|
| * Reference only | | | | |

As is clearly understood from Table 2, according to the present invention, by using a gene that codes a specific kind of enzyme in a gene that codes (R)-3-hydroxybutyryl-CoA selective acetoacetyl-CoA reductase and a gene that codes (R)-3-hydroxybutyryl-CoA selective enoyl-CoA hydratase, 1,4-butanediol can be efficiently manufactured.

## Claims

1. A method of manufacturing 1,4-butanediol, using a microbe and/or a culture thereof, by an enzyme reaction system that uses acetoacetyl-CoA reductase and enoyl-CoA hydratase, via acetoacetyl-CoA, 3-hydroxybutyryl-CoA and crotonyl-CoA in this order,
wherein the acetoacetyl-CoA reductase is (R)-3-hydroxybutyryl-CoA selective acetoacetyl-CoA reductase and the enoyl CoA reductase is (R)-3-hydroxybutyryl-CoA selective enoyl-CoA hydratase, and
wherein the microbe includes
(1) a gene that codes the (R)-3-hydroxybutyryl-CoA selective acetoacetyl-CoA reductase, and
(2) a gene that codes the (R)-3-hydroxybutyryl-CoA selective enoyl-CoA hydratase.

2. The method of manufacturing 1,4-butanediol according to claim 1,
wherein the (R)-3-hydroxybutyryl-CoA selective acetoacetyl-CoA reductase is acetoacetyl-CoA reductase (EC number: 1.1.1.36), and
wherein the (R)-3-hydroxybutyryl-CoA selective enoyl-CoA hydratase is enoyl-CoA hydratase (EC number: 4.2.1.55 or EC number: 4.2.1.119).

3. The method of manufacturing 1,4-butanediol according to claim 1,
wherein a gene that codes the (R)-3-hydroxybutyryl-CoA selective acetoacetyl-CoA reductase is any one of undermentioned genes (a) to (c), and
wherein a gene that codes the (R)-3-hydroxybutyryl-CoA selective enoyl-CoA hydratase is any one of undermentioned genes (d) to (f):
(a) a gene that has a base sequence of sequence number 3;
(b) a gene that has a base sequence such that one or more bases are deleted, substituted, or added in a base sequence of sequence number 3, wherein the gene has a base sequence with an identity greater than or equal to 90% with respect to the base sequence of sequence number 3;
(c) a gene that hybridizes with a gene that has a base sequence complementary with a gene that has a base sequence described in sequence number 3 on a stringent condition;
(d) a gene that has a base sequence of sequence number 5;
(e) a gene that has a base sequence such that one or more bases are deleted, substituted, or added in a base sequence of sequence number 5, wherein the gene has a base sequence with an identity greater than or equal to 90% with respect to the base sequence of sequence number 5;
(f) a gene that hybridizes with a gene that has a base sequence complementary with a gene that has a base sequence described in sequence number 5 on a stringent condition.

4. The method of manufacturing 1,4-butanediol according to claim 1,
wherein the microbe further includes,
(1) a gene that codes vinylacetyl-CoA delta-isomerase (EC number: 5.3.3.3),
(2) a gene that codes 4-hydroxybutyryl-CoA dehydratase (EC number: 4.2.1.120), and/or
(3) a gene that codes acyl-CoA reductase (EC number: 1.2.1.10).

5. The method of manufacturing 1,4-butanediol according to claim 1,
wherein the microbe is Escherichia coli, a yeast, a corynebacterium or a clostridial bacteria.

6. A microbe that manufactures 1,4-butanediol by an enzyme reaction system that uses acetoacetyl-CoA reductase and enoyl-CoA hydratase, via acetoacetyl-CoA, 3-hydroxybutyryl-CoA and crotonyl-CoA in this order,
wherein the acetoacetyl-CoA reductase is (R)-3-hydroxybutyryl-CoA selective acetoacetyl-CoA reductase and the enoyl CoA reductase is (R)-3-hydroxybutyryl-CoA selective enoyl-CoA hydratase, and
wherein the microbe includes
(1) a gene that codes the (R)-3-hydroxybutyryl-CoA selective acetoacetyl-CoA reductase, and
(2) a gene that codes the (R)-3-hydroxybutyryl-CoA selective enoyl-CoA hydratase.

7. The microbe according to claim 6,
wherein the microbe is Escherichia coli, a yeast, a corynebacterium or a clostridial bacteria.

## Patentansprüche

1. Verfahren zum Herstellen von 1,4-Butandiol unter Einsatz einer Mikrobe und/oder einer Kultur davon durch ein Acetoacetyl-CoA-Reduktase und Enoyl-CoA-Hydratase verwendendes Enzymreaktionssystem über Acetoacetyl-CoA, 3-Hydroxybutyryl-CoA und Crotonyl-CoA in dieser Reihenfolge,
wobei die Acetoacetyl-CoA-Reduktase eine für (R)-3-Hydroxybutyryl-CoA selektive Acetoacetyl-CoA-Reduktase ist und die Enoyl-CoA-Reduktase eine für (R)-3-Hydroxybuyryl-CoA selektive Enoyl-CoA-Hydratase ist, und
wobei die Mikrobe
(1) ein Gen, das für eine für (R)-3-Hydroxybutyryl-CoA selektive Acetoacetyl-CoA-Reduktase kodiert, und
(2) ein Gen umfasst, das für eine für (R)-3-Hydroxybutyryl-CoA selektive Enoyl-CoA-Hydratase kodiert.

2. Verfahren zum Herstellen von 1,4-Butandiol nach Anspruch 1,
wobei die für (R)-3-Hydroxybutyryl-CoA selektive Acetoacetyl-CoA-Reduktase Acetoacetyl-CoA-Reduktase (EC Nummer: 1.1.1.36) ist und
wobei die für die (R)-3-Hydroxybutyryl-CoA selektive Enoyl-CoA-Hydratase Enoyl-CoA-Hydratase (EC Nummer: 4.2.1.55 oder EC Nummer: 4.2.1.119) ist.

3. Verfahren zum Herstellen von 1,4-Butandiol nach Anspruch 1,
wobei ein Gen, das für die für (R)-3-Hydroxybutyryl-CoA selektive Acetoacetyl-CoA-Reduktase kodiert, ein beliebiges der nachstehend genannten Gene (a) bis (c) ist und
wobei ein Gen, das für ein für die (R)-3-Hydroxybutyryl-CoA selektive Enoyl-CoA-Hydratase kodiert, ein beliebiges der nachstehend genannten Gene (d) bis (f) ist:
(a) ein Gen, das die Basensequenz der Sequenz ID Nummer 3 aufweist;
(b) ein Gen, das eine Basensequenz aufweist, so dass in einer Basensequenz der Sequenz ID Nummer 3 eine oder mehrere Basen deletiert, substituiert oder addiert sind, wobei das Gen eine Basensequenz mit einer Identität zu der Basensequenz der Sequenz ID Nummer 3 von größer oder gleich 90 % aufweist;
(c) ein Gen, das mit einem Gen, das eine Basensequenz aufweist, die zu einem die in Sequenz ID Nummer 3 beschriebene Basensequenz aufweisenden Gen komplementär ist, unter einer stringenten Bedingung hybridisiert;
(d) ein Gen, das eine Basensequenz der Sequenz ID Nummer 5 aufweist;
(e) ein Gen, das eine Basensequenz aufweist, so dass eine oder mehrere Basen in einer Basensequenz der Sequenznummer 5 deletiert, substituiert oder addiert sind, wobei das Gen eine Basensequenz mit einer Identität zu der Basensequenz der Sequenz ID Nummer 5 von größer oder gleich 90 % aufweist;
(f) ein Gen, das mit einem Gen einer Basensequenz, die zu einem die in Sequenz ID Nummer 5 beschriebene Basensequenz aufweisenden Gen komplementär ist, unter einer Stringenten Bedingung hybridisiert.

4. Verfahren zum Herstellen von 1,4-Butandiol nach Anspruch 1,
wobei die Mikrobe außerdem
(1) ein Gen, das für Vinylacetyl-CoA-Delta-Isomerase (EC Nummer: 5.3.3.3) kodiert,
(2) ein Gen, das für 4-Hydroxybutyryl-CoA-Dehydratase (EC Nummer: 4.2.1.120) kodiert und/oder
(3) ein Gen umfasst, das für Acyl-CoA-Reduktase (EC Nummer: 1.2.1.10) kodiert.

5. Verfahren zum Herstellen von 1,4-Butandiol nach Anspruch 1, wobei die Mikrobe Escherichia coli, eine Hefe, ein Corynebacterium oder ein Clostridiumbacterium ist.

6. Mikrobe, die 1,4-Butandiol durch ein Acetoacetyl-CoA-Reduktase und Enoyl-CoA-Hydratase verwendendes Enzymreaktionssystem über Acetoacetyl-CoA, 3-Hydroxybutyryl-CoA und Crotonyl-CoA in dieser Reihenfolge herstellt,
wobei die Acetoacetyl-CoA-Reduktase eine für (R)-3-Hydroxybutyryl-CoA selektive Acetoacetyl-CoA-Reduktase ist und die Enoyl-CoA-Reduktase eine für (R)-3-Hydroxybutyryl-CoA selektive Enoyl-CoA-Hydratase ist und
wobei die Mikrobe
(1) ein Gen, das für eine für (R)-3-Hydroxybutyryl-CoA selektive Acetoacetyl-CoA-Reduktase kodiert, und
(2) ein Gen umfasst, das für eine für (R)-3-Hydroxybutyryl-CoA selektive Enyoyl-CoA-Hydratase kodiert.

7. Mikrobe nach Anspruch 6, wobei die Mikrobe Escherichia coli, eine Hefe, ein Corynebacterium oder ein Clostridiumbacterium ist.

## Revendications

1. Procédé de fabrication de 1,4-butanediol, faisant appel à un microbe et/ou à une culture de celui-ci, par un système de réaction enzymatique mettant en oeuvre une acétoacétyle-CoA réductase et une énoyle-CoA hydratase, par l'intermédiaire, dans cet ordre, de : acétoacétyle-CoA, 3-hydroxybutyryle-CoA et crotonyle-CoA,
dans lequel l'acétoacétyle-CoA réductase est l'acétoacétyle-CoA réductase spécifique à (R)-3-hydroxybutyryle-CoA et l'énoyle-CoA réductase est l'énoyle-CoA hydratase spécifique à (R)-3-hydroxybutyryle-CoA, et
dans lequel le microbe inclut
(1) un gène qui code pour l'acétoacétyle-CoA réductase spécifique à (R)-3-hydroxybutyryle-CoA, et
(2) un gène qui code pour l'énoyle-CoA hydratase spécifique à (R)-3-hydroxybutyryle-CoA.

2. Procédé de fabrication de 1,4-butanediol selon la revendication 1,
dans lequel l'acétoacétyle-CoA réductase spécifique à (R)-3-hydroxybutyryle-CoA est l'acétoacétyle-CoA réductase (numéro CE : 1.1.1.36), et
dans lequel l'énoyle-CoA hydratase spécifique à (R)-3-hydroxybutyryle-CoA est l'énoyle-CoA hydratase (numéro CE : 4.2.1.55 ou numéro CE : 4.2.1.119).

3. Procédé de fabrication de 1,4-butanediol selon la revendication 1,
dans lequel un gène qui code pour l'acétoacétyle-CoA réductase spécifique à (R)-3-hydroxybutyryle-CoA est l'un quelconque des gènes (a) à (c) mentionnés ci-dessous, et
dans lequel un gène qui code pour l'énoyle-CoA hydratase spécifique à (R)-3-hydroxybutyryle-CoA est l'un quelconque des gènes (d) à (f) mentionnés ci-dessous :
(a) un gène qui comporte une séquence de base de numéro de séquence 3 ;
(b) un gène qui comporte une séquence de base telle qu'une ou plusieurs bases sont supprimées, substituées ou ajoutées dans une séquence de base de numéro de séquence 3, le gène ayant une séquence de base dont l'identité est supérieure ou égale à 90 % par rapport à la séquence de base de numéro de séquence 3 ;
(c) un gène qui s'hybride avec un gène qui comporte une séquence de base complémentaire avec un gène qui comporte une séquence de base décrite dans le numéro de séquence 3 dans des conditions strictes ;
(d) un gène qui comporte une séquence de base de numéro de séquence 5 ;
(e) un gène qui comporte une séquence de base telle qu'une ou plusieurs bases sont supprimées, substituées ou ajoutées dans une séquence de base de numéro de séquence 5, le gène ayant une séquence de base dont l'identité est supérieure ou égale à 90 % par rapport à la séquence de base de numéro de séquence 5 ;
(f) un gène qui s'hybride avec un gène qui comporte une séquence de base complémentaire avec un gène qui comporte une séquence de base décrite dans le numéro de séquence 5 dans des conditions strictes.

4. Procédé de fabrication de 1,4-butanediol selon la revendication 1,
dans lequel le microbe inclut en outre,
(1) un gène qui code pour la vinylacétyle-CoA delta-isomérase (numéro CE : 5.3.3.3),
(2) un gène qui code pour la 4-hydroxybutyryle-CoA déshydratase (numéro CE : 4.2.1.120), et/ou
(3) un gène qui code pour l'acyle-CoA réductase (numéro CE : 1.2.1.10).

5. Procédé de fabrication de 1,4-butanediol selon la revendication 1,
dans lequel le microbe est Escherichia coli, une levure, un corynebacterium ou une bactérie clostridienne.

6. Microbe qui fabrique du 1,4-butanediol par un système de réaction enzymatique mettant en oeuvre une acétoacétyle-CoA réductase et une énoyle-CoA hydratase, par l'intermédiaire, dans cet ordre, de : acétoacétyle-CoA, 3-hydroxybutyryle-CoA et crotonyle-CoA,
dans lequel l'acétoacétyle-CoA réductase est l'acétoacétyle-CoA réductase spécifique à (R)-3-hydroxybutyryle-CoA et l'énoyle-CoA réductase est l'énoyle-CoA hydratase spécifique à (R)-3-hydroxybutyryle-CoA, et
dans lequel le microbe inclut
(1) un gène qui code pour l'acétoacétyle-CoA réductase spécifique à (R)-3-hydroxybutyryle-CoA, et
(2) un gène qui code pour l'énoyle-CoA hydratase spécifique à (R)-3-hydroxybutyryle-CoA.

7. Microbe selon la revendication 6,
dans lequel le microbe est Escherichia coli, une levure, un corynebacterium ou une bactérie clostridienne.


### SEQUENCE LISTING

<110> Showa Denko K.K.
   <120> Method of producing 1,4-butanediol and microorganism
   <130> P20120217 (SHD13009PCT)
   <160> 7
   <170> PatentIn version 3.5
<210> 1
   <211> 1185
   <212> DNA
   <213> Artificial Sequence
   <220>
   <223> Escherichia coli atoB gene
<400> 1
   atgaaaaatt gtgtcatcgt cagtgcggta cgtactgcta tcggtagttt taacggttca 60
   ctcgcttcca ccagcgccat cgacctgggg gcgacagtaa ttaaagccgc cattgaacgt 120
   gcaaaaatcg attcacaaca cgttgatgaa gtgattatgg gtaacgtgtt acaagccggg 180
   ctggggcaaa atccggcgcg tcaggcactg ttaaaaagcg ggctggcaga aacggtgtgc 240
   ggattcacgg tcaataaagt atgtggttcg ggtcttaaaa gtgtggcgct tgccgcccag 300
   gccattcagg caggtcaggc gcagagcatt gtggcggggg gtatggaaaa tatgagttta 360
   gccccctact tactcgatgc aaaagcacgc tctggttatc gtcttggaga cggacaggtt 420
   tatgacgtaa tcctgcgcga tggcctgatg tgcgccaccc atggttatca tatggggatt 480
   accgccgaaa acgtggctaa agagtacgga attacccgtg aaatgcagga tgaactggcg 540
   ctacattcac agcgtaaagc ggcagccgca attgagtccg gtgcttttac agccgaaatc 600
   gtcccggtaa atgttgtcac tcgaaagaaa accttcgtct tcagtcaaga cgaattcccg 660
   aaagcgaatt caacggctga agcgttaggt gcattgcgcc cggccttcga taaagcagga 720
   acagtcaccg ctgggaacgc gtctggtatt aacgacggtg ctgccgctct ggtgattatg 780
   gaagaatctg cggcgctggc agcaggcctt acccccctgg ctcgcattaa aagttatgcc 840
   agcggtggcg tgccccccgc attgatgggt atggggccag tacctgccac gcaaaaagcg 900
   ttacaactgg cggggctgca actggcggat attgatctca ttgaggctaa tgaagcattt 960
   gctgcacagt tccttgccgt tgggaaaaac ctgggctttg attctgagaa agtgaatgtc 1020
   aacggcgggg ccatcgcgct cgggcatcct atcggtgcca gtggtgctcg tattctggtc 1080
   acactattac atgccatgca ggcacgcgat aaaacgctgg ggctggcaac actgtgcatt 1140
   ggcggcggtc agggaattgc gatggtgatt gaacggttga attaa 1185
<210> 2
   <211> 849
   <212> DNA
   <213> Artificial Sequence
   <220>
   <223> Clostridium kluyveri hbd gene
   <400> 2
   atgagtatta aaagtgtagc ggttttaggt agtggaacta tgtctcgtgg aattgtgcag 60
   gcttttgcag aagcaggtat agatgtaatt atccgtggaa gaactgaagg tagtattgga 120
   aaaggtctag cagcagtaaa gaaagcttat gataaaaaag tatcaaaggg gaaaatttcc 180
   caggaagatg ctgataaaat agttggaaga gtaagtacaa caactgaact tgaaaaattg 240
   gctgattgtg atcttataat agaagcagca tcagaggata tgaatataaa gaaagactat 300
   tttggaaaat tagaagaaat atgcaagcct gaaacaattt ttgctactaa tacttcttca 360
   ttatctataa ctgaagtagc aacagctaca aagagaccag ataaattcat aggaatgcat 420
   ttctttaatc cagcaaatgt tatgaaatta gttgaaatca taagaggtat gaatacttca 480
   caagaaactt ttgatattat aaaagaagct tccattaaaa taggaaaaac tcctgtagaa 540
   gttgcagaag ctccaggatt tgttgtaaac aagatattag taccaatgat caatgaagca 600
   gtaggaattt tggcagaagg aatagcttca gcagaagata tcgatacagc tatgaaatta 660
   ggcgctaatc acccaatggg tcctttagca ttaggagatc ttattggact tgatgtagtt 720
   cttgcagtta tggatgtact ttatagtgaa actggagatt caaaatatag agctcataca 780
   ttacttagaa aatatgtaag agcaggatgg cttggaagaa aatcaggaaa aggattcttc 840
   gcttattaa 849
<210> 3
   <211> 741
   <212> DNA
   <213> Artificial Sequence
   <220>
   <223> Ralstonia eutropha phaB gene
   <400> 3
   atgactcagc gcattgcgta tgtgaccggc ggcatgggtg gtatcggaac cgccatttgc 60
   cagcggctgg ccaaggatgg ctttcgtgtg gtggccggtt gcggccccaa ctcgccgcgc 120
   cgcgaaaagt ggctggagca gcagaaggcc ctgggcttcg atttcattgc ctcggaaggc 180
   aatgtggctg actgggactc gaccaagacc gcattcgaca aggtcaagtc cgaggtcggc 240
   gaggttgatg tgctgatcaa caacgccggt atcacccgcg acgtggtgtt ccgcaagatg 300
   acccgcgccg actgggatgc ggtgatcgac accaacctga cctcgctgtt caacgtcacc 360
   aagcaggtga tcgacggcat ggccgaccgt ggctggggcc gcatcgtcaa catctcgtcg 420
   gtgaacgggc agaagggcca gttcggccag accaactact ccaccgccaa ggccggcctg 480
   catggcttca ccatggcact ggcgcaggaa gtggcgacca agggcgtgac cgtcaacacg 540
   gtctctccgg gctatatcgc caccgacatg gtcaaggcga tccgccagga cgtgctcgac 600
   aagatcgtcg cgacgatccc ggtcaagcgc ctgggcctgc cggaagagat cgcctcgatc 660
   tgcgcctggt tgtcgtcgga ggagtccggt ttctcgaccg gcgccgactt ctcgctcaac 720
   ggcggcctgc atatgggctg a 741
<210> 4
   <211> 786
   <212> DNA
   <213> Artificial Sequence
   <220>
   <223> Clostridium acetobutylicum crt gene
   <400> 4
   atggaactaa acaatgtcat ccttgaaaag gaaggtaaag ttgctgtagt taccattaac 60
   agacctaaag cattaaatgc gttaaatagt gatacactaa aagaaatgga ttatgttata 120
   ggtgaaattg aaaatgatag cgaagtactt gcagtaattt taactggagc aggagaaaaa 180
   tcatttgtag caggagcaga tatttctgag atgaaggaaa tgaataccat tgaaggtaga 240
   aaattcggga tacttggaaa taaagtgttt agaagattag aacttcttga aaagcctgta 300
   atagcagctg ttaatggttt tgctttagga ggcggatgcg aaatagctat gtcttgtgat 360
   ataagaatag cttcaagcaa cgcaagattt ggtcaaccag aagtaggtct cggaataaca 420
   cctggttttg gtggtacaca aagactttca agattagttg gaatgggcat ggcaaagcag 480
   cttatattta ctgcacaaaa tataaaggca gatgaagcat taagaatcgg acttgtaaat 540
   aaggtagtag aacctagtga attaatgaat acagcaaaag aaattgcaaa caaaattgtg 600
   agcaatgctc cagtagctgt taagttaagc aaacaggcta ttaatagagg aatgcagtgt 660
   gatattgata ctgctttagc atttgaatca gaagcatttg gagaatgctt ttcaacagag 720
   gatcaaaagg atgcaatgac agctttcata gagaaaagaa aaattgaagg cttcaaaaat 780
   agatag 786
<210> 5
   <211> 405
   <212> DNA
   <213> Artificial Sequence
   <220>
   <223> Aeromonas caviae phaJ gene
   <400> 5
   atgagcgcgc agagcctgga agtgggtcaa aaagctcgcc tgtccaaacg ttttggtgca
   60
   gcagaagttg cggccttcgc agcactgtct gaagacttca atccgctgca tctggacccg
   120
   gcattcgcgg caaccaccgc atttgaacgc ccgatcgttc atggtatgct gttggcaagc
   180
   ctgtttagcg gtctgctggg tcagcagctg ccgggcaaag gtagcattta cctgggtcag
   240
   agcctgagct ttaaactgcc ggtgttcgtc ggtgacgagg tcacggcgga ggtcgaggtt
   300
   acggccctgc gtgaggacaa gccgatcgcg accctgacca cccgtatttt cacccagggt
   360
   ggcgcgttgg ccgtgacggg tgaggccgtg gtcaaactgc cgtaa                    405
<210> 6
   <211> 1473
   <212> DNA
   <213> Artificial Sequence
   <220>
   <223> putative gene encoding enzyme converting crotonyl-CoA to 4-hydroxybutyryl-CoA
   <400> 6
   atgctgatga ccgccgaaca gtacatcgaa agcctgcgca agctgaacac gcgtgtctac 60
   atgtttggtg aaaagatcga gaactgggta gaccatccca tgattcgccc ttcgatcaat 120
   tgtgtggcaa tgacctatga actggcgcaa gatccccagt atgccgattt gatgaccacg 180
   aaatcgaatc tgattggcaa gacgattaat cgcttcgcga acttacacca aagcaccgat 240
   gatctgcgca agaaagtcaa aatgcaacgt ctgctgggcc agaaaaccgc gagttgcttt 300
   cagcggtgtg ttgggatgga tgcgttcaat gccgtgtttt ccaccactta tgagattgat 360
   cagaaatacg ggactaacta ccacaagaac ttcacggagt atctgaaata tattcaagaa 420
   aatgacttga tcgtggatgg tgctatgacc gacccgaaag gcgaccgtgg gctggcaccg 480
   agtgcccaga aagacccaga tctgtttctg cgcatcgtgg agaaacgcga ggatggtatc 540
   gtagtccgtg gtgcgaaagc acatcaaacg ggctcaatta atagccatga gcacatcatt 600
   atgccgacca ttgctatgac agaagcggat aaagactatg cggttagttt tgcttgtcca 660
   tccgatgcgg atggtttgtt tatgatctat ggtcgccagt cttgcgacac ccgcaaaatg 720
   gaggaaggcg cagatattga cttaggcaac aaacagtttg gcggccaaga agcccttgtc 780
   gtgtttgaca acgtattcat cccgaatgat cgcattttct tatgccagga atacgatttt 840
   gcgggaatga tggtcgaacg cttcgctggt tatcatcgcc agtcttacgg tggctgcaaa 900
   gtgggtgtgg gtgacgtcgt tattggagcc gcagcccttg cagcggatta caacggtgcc 960
   cagaaggcca gccatgtgaa agacaaactg atcgaaatga cacacttgaa cgaaaccctg 1020
   tattgttgtg ggattgcgtg ctcagccgaa ggttatccga ctgcagccgg caattaccag
   1080
   atcgatctcc tccttgcgaa tgtgtgcaaa cagaacatta cgcgttttcc gtacgagatt
   1140
   gtacgtctgg cggaagatat tgccggagga ctgatggtta ctatgcctag cgaagcggac
   1200
   ttcaaaagcg aaacggttgt tgggcgtgat ggcgaaacca ttggcgattt ctgcaacaaa
   1260
   ttctttgcgg cagcaccgac ttgcaccaca gaagaacgga tgcgtgtgct ccgctttctg
   1320
   gagaacattt gtttaggcgc atccgctgtt gggtatcgta cagaatcgat gcatggtgcg
   1380
   ggctctccac aggctcaacg catcatgatt gcccgtcaag gcaatattaa cgcgaagaaa
   1440
   gaactcgcta aagcgatcgc tggcatcaaa taa                              1473
<210> 7
   <211> 1407
   <212> DNA
   <213> Artificial Sequence
   <220>
   <223> Clostridium beijerinckii ald gene
   <400> 7
   atgaataaag acacactaat acctacaact aaagatttaa aagtaaaaac aaatggtgaa 60
   aacattaatt taaagaacta caaggataat tcttcatgtt tcggagtatt cgaaaatgtt 120
   gaaaatgcta taagcagcgc tgtacacgca caaaagatat tatcccttca ttatacaaaa 180
   gagcaaagag aaaaaatcat aactgagata agaaaggccg cattacaaaa taaagaggtc 240
   ttggctacaa tgattctaga agaaacacat atgggaagat atgaggataa aatattaaaa 300
   catgaattgg tagctaaata tactcctggt acagaagatt taactactac tgcttggtca 360
   ggtgataatg gtcttacagt tgtagaaatg tctccatatg gtgttatagg tgcaataact 420
   ccttctacga atccaactga aactgtaata tgtaatagca taggcatgat agctgctgga 480
   aatgctgtag tatttaacgg acacccatgc gctaaaaaat gtgttgcctt tgctgttgaa 540
   atgataaata aggcaattat ttcatgtggc ggtcctgaaa atctagtaac aactataaaa 600
   aatccaacta tggagtctct agatgcaatt attaagcatc cttcaataaa acttctttgc 660
   ggaactgggg gtccaggaat ggtaaaaacc ctcttaaatt ctggtaagaa agctataggt
   720
   gctggtgctg gaaatccacc agttattgta gatgatactg ctgatataga aaaggctggt
   780
   aggagcatca ttgaaggctg ttcttttgat aataatttac cttgtattgc agaaaaagaa
   840
   gtatttgttt ttgagaatgt tgcagatgat ttaatatcta acatgctaaa aaataatgct
   900
   gtaattataa atgaagatca agtatcaaaa ttaatagatt tagtattaca aaaaaataat
   960
   gaaactcaag aatactttat aaacaaaaaa tgggtaggaa aagatgcaaa attattctta
   1020
   gatgaaatag atgttgagtc tccttcaaat gttaaatgca taatctgcga agtaaatgca
   1080
   aatcatccat ttgttatgac agaactcatg atgccaatat tgccaattgt aagagttaaa
   1140
   gatatagatg aagctattaa atatgcaaag atagcagaac aaaatagaaa acatagtgcc
   1200
   tatatttatt ctaaaaatat agacaaccta aatagatttg aaagagaaat agatactact
   1260
   atttttgtaa agaatgctaa atcttttgct ggtgttggtt atgaagcaga aggatttaca
   1320
   actttcacta ttgctggatc tactggtgag ggaataacct ctgcaaggaa ttttacaaga
   1380
   caaagaagat gtgtacttgc cggctaa                                      1407